# EUROPEAN PATENT APPLICATION

(11) **EP 4 763 111 A1**
(43) Date of publication of application: **24.06.2026**
(21) Application number: 25221890.4
(22) Date of filing: 09.12.2025
(51) Int. Cl.: A61B 17/70

(54) **COUPLING DEVICE FOR COUPLING A ROD TO A BONE ANCHORING ELEMENT**

(30) Priority: 23.12.2024 US 202463737974 P; 23.12.2024 EP 24223030
(71) Applicant: Biedermann Technologies GmbH & Co. KG, 78166 Donaueschingen (DE)
(72) Inventor: BIEDERMANN, Lutz, 78048 VS-Villingen (DE); BIEDERMANN, Timo, 78647 Trossingen (DE); DANNECKER, Berthold, 78112 St. Georgen (DE)
(74) Representative: Prüfer & Partner mbB Patentanwälte · Rechtsanwälte

(57) **Abstract**

A coupling device for coupling a rod (100) to a bone anchoring element (1) having a head (3) and a shank (2) comprises a receiving part (5) having a first end (5a), a second end (5b) and a passage (51) extending from the first end (5a) to the second end (5b), a recess (54) for receiving the rod (100), the recess (54) extending from the first end (5a) towards the second end (5b) and forming two free legs (55) and an accommodation space (53) for accommodating the head (3) of the bone anchoring element (1), the accommodation space (53) having an opening (52) at the second end (5b), a pressure element (6) configured to be located at least partially in the accommodation space (53) and comprising a first portion (61) comprising a rod support surface (62a) and a second portion (66) having a hollow interior (67) with an opening defining a flexible portion configured to accommodate and clamp the head (3), wherein the receiving part (5) comprises a first securing portion (58a) and the pressure element (6) comprises a second securing portion (63a) configured to engage the first securing portion (58a), wherein the pressure element (6) is insertable into the receiving part (5) from the second end (5b) in a first rotational orientation relative to the receiving part (5) in which the first securing portion and the second securing portion are out of engagement and wherein the pressure element (6) is rotatable in the receiving part (5) to assume a second rotational orientation with respect to the receiving part (5) in which the first securing portion (58a) and the second securing portion (63a) are engaged to prevent the pressure element (6) from moving towards the first end (5a).

## Description

The invention relates to a coupling device for coupling a rod to a bone anchoring element. In particular, the invention relates to a coupling device that forms part of a polyaxial bone anchoring device.

Various types of polyaxial bone anchoring devices are known in the art. Usually, a polyaxial bone anchoring device includes a coupling device and a bone anchoring element with a head that is pivotably received in the coupling device and can be locked at a desired angle of the bone anchoring element relative to the coupling device. The coupling device also receives a rod that is configured to connect the polyaxial bone anchoring device to a further bone anchor.

For example, US 9,486,246 B2 describes a bone anchoring device that includes an anchoring element including a shaft and a head; a receiving part having a first end and a second end and a channel for receiving a rod near the first end, an accommodation space for accommodating the head near the second end and a pressure element configured to be located at least partially in the accommodation space and including a flexible portion to clamp the head. The coupling device may be assembled by inserting the pressure element from the first end into the receiving part in a first rotational orientation, then the pressure element is rotated by 90° such that a projection engages a recessed portion in the receiving part. In this position, the pressure element is prevented from escaping through the first end. The bone anchoring device can be used as a bottom loading polyaxial bone anchoring device, wherein the bone anchoring element is inserted into the receiving part from the second or bottom end. In an alternative way of assembling, the pressure element is first assembled with the bone anchoring element. Then the bone anchoring element with the pressure element mounted thereon is inserted from the first or top end into the receiving part in a first rotational orientation and then rotated until a projection of the pressure element engages the recess section of the receiving part in a second rotational orientation.

It is an object of the present invention to provide an improved coupling device that can be used with a bone anchoring element having an enlarged head and a bone anchoring device including such a bone anchoring element. The object is solved by a coupling device according to claim 1 and by a method of assembling the coupling device according to claim 17. Further developments are given in the dependent claims.

According to an embodiment a coupling device for coupling a rod to a bone anchoring element having a head and a shank includes a receiving part having a first end, a second end, a central axis extending between the first end and the second end and a passage extending from the first end to the second end, a recess for receiving the rod, the recess extending the from the first end towards the second end and forming two free legs and an accommodation space for accommodating the head of the bone anchoring element, the accommodation space having an opening at the second end. The coupling device further includes a pressure element configured to be located at least partially in the accommodation space and comprising a first portion comprising a rod support surface and a second portion having a hollow interior with an opening defining a flexible portion configured to accommodate and clamp the head. The receiving part further comprises a first securing portion and the pressure element comprises a second securing portion configured to engage the first securing portion, wherein the pressure element is insertable into the receiving part from the second end in a first rotational orientation relative to the receiving part in which the first securing portion and the second securing portion are out of engagement and wherein the pressure element is rotatable in the receiving part to assume a second rotational orientation with respect to the receiving part in which the first securing portion and the second securing portion are engaged to prevent the pressure element from moving towards the first end.

With the coupling device bone anchoring elements can be used that have an enlarged head that cannot be inserted through the first or top end of the coupling device. Instead, the bone anchoring device is designed as a bottom loading bone anchoring device in which the head of the bone anchoring element is inserted into the receiving part from the bottom end thereof.

Due to the design of the coupling device, the overall lateral dimension of the coupling device may be kept small. At the same time, a robust bone anchoring device can be provided due to an increased holding force between the head of the bone anchoring element in the pressure element and the receiving part.

In addition, a maximum pivot angle of the bone anchoring element relative to the receiving part may be increased.

The coupling device and the bone anchoring device including such a coupling device provides a modular bone anchoring device wherein the parts can be selected and assembled in a simple manner at the operation site, or at any other place after the parts have been manufactured. Such a modular bone anchoring device allows for combinations of various anchoring elements with the receiving part on demand, depending on actual clinical requirements and situations. This reduces the costs of the bone anchoring devices, reduces the inventory, gives the surgeon a substantial choice of implants.

Further features and advantages of the invention will become apparent from the description of embodiments by means of the accompanying drawings. In the drawings:
- Fig. 1:: shows a perspective exploded view of a polyaxial bone anchoring device with a coupling device and a rod according to a first embodiment.
- Fig. 2:: shows a perspective view of the polyaxial bone anchoring device of Fig. 1 in the assembled state.
- Fig. 3:: shows a cross-sectional view of the polyaxial bone anchoring device of Fig. 2, the cross-section taken in a plane perpendicular to the rod axis of the inserted rod.
- Fig. 4:: shows a perspective view from a top of the receiving part of the coupling device according to Figs. 1 to 3.
- Fig. 5:: shows a perspective view from a bottom of the receiving part of Fig. 4.
- Fig. 6:: shows a top view of the receiving part of Figs. 4 and 5.
- Fig. 7:: shows a cross-sectional view of the receiving part of Figs. 4 to 6, the cross-section taken along line A-A in Fig. 6.
- Fig. 8:: shows a perspective view from a top of a pressure element of the coupling device according to Figs. 1 to 3.
- Fig. 9:: shows a perspective view from a bottom of the pressure element of Fig. 8.
- Fig. 10:: shows a top view of the pressure element of Figs. 8 and 9.
- Fig. 11a:: shows a cross-sectional view of the pressure element of Figs. 8 to 10, the cross-section taken along line B-B in Fig. 10.
- Fig. 11b:: shows an enlarged portion of Fig. 11a.
- Figs. 12 to 15:: show perspective views of steps of assembling the receiving part and the pressure element of the coupling device of Figs. 1 to 3.
- Figs. 16 to 19:: show cross-sectional views of steps of assembling the polyaxial bone anchoring device, the cross-section taken in a plane perpendicular to the axis of the channel for the rod.

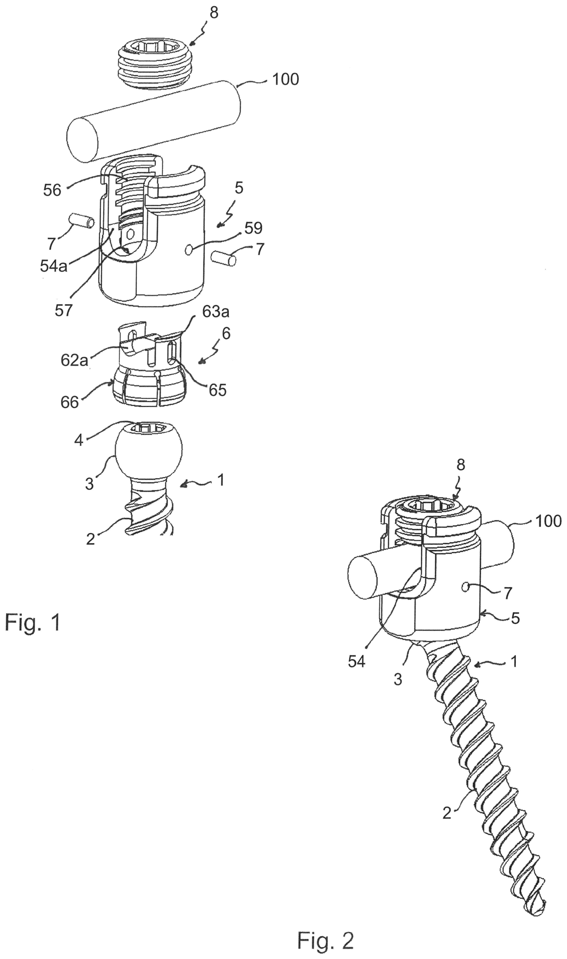

Referring to Figs. 1 to 3, the bone anchoring device includes a bone anchoring element 1 having a shank 2 for anchoring in bone or in a vertebra and a head 3. The shank 2 has a bone engagement feature, such as a thread. The head 3 is shaped as a segment of a sphere, thus comprises a spherically-shaped outer surface portion. The spherically-shaped outer surface portion may include a region with a greatest outer diameter of the head 3. In addition, the head 3 comprises at its free end an engagement portion 4 for engagement with a tool, such as a driver. Further, the bone anchoring device includes a receiving part 5 for receiving the head 3 of the bone anchoring element 1 and for receiving a rod 100 which is configured to connect several bone anchoring devices. In the receiving part 5, a pressure element 6 is provided for exerting pressure onto the head 3 of the bone anchoring element 1 and for providing support for the rod 100. The pressure element 6 may be secured by pins 7. To lock the head and the rod 100 in the receiving part 5, a locking element 8 in the form of an inner screw or set screw is provided.

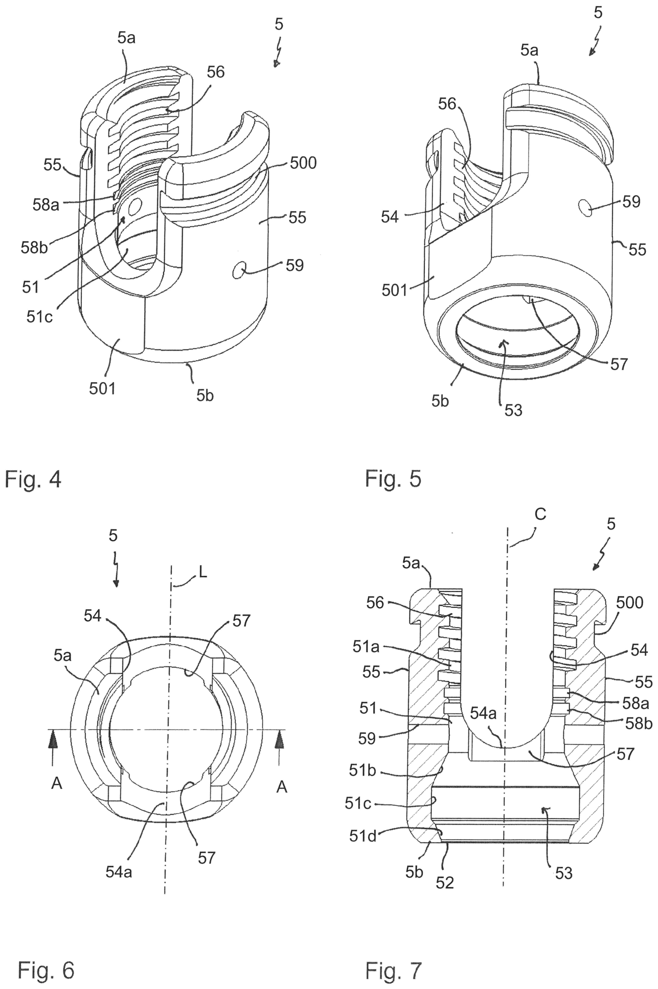

Referring in addition to Figs. 4 to 7, the receiving part will be explained in greater detail. The receiving part 5 has a first or top end 5a and a second or bottom end 5b opposite to the top end 5a. In general, the receiving part 5 may have a substantially cylindrical outer shape with a central longitudinal axis C extending through the top end 5a and the bottom end 5b. Coaxially to the central axis C, a passage 51 is provided that extends from the top end 5a to the bottom end 5b and that forms an opening 52 at the bottom end 5b. At a distance from the top end 5a, the passage 51 widens into an accommodation space 53 that is configured to receive the head 3 and at least a portion of the pressure element 6 therein. The receiving part 5 further has a substantially U-shaped recess 54 starting at the top end 5a and extending in a direction of the bottom end 5b. By means of the U-shaped recess 54 two free legs 55 are formed and define a channel that is open towards the first end 5a for receiving the rod 100. On an inner surface of the legs 55, an internal thread 56 is formed, which is in the exemplary embodiment a square thread or another flat thread.

As can be seen in particular in Fig. 7, a portion of the passage 51 starting from the top end 5a is formed by a substantially cylindrical bore 51a with an inner diameter that is smaller than a greatest outer diameter of the head 3. In particular, the greatest outer diameter of the head 3 may be the same or may be greater than a core diameter of the internal thread 56. The bore 51a of the passage 51 may extend below the bottom 54a of the U-shaped recess 54 in an axial direction. Following the bore 51a, the passage widens in a widening portion 51b into a substantially cylindrical portion 51c that has an inner diameter greater than a greatest outer diameter of the head 3 and greater than an outer diameter of the pressure element 6 with the inserted head 3. Following the cylindrical section 51c, the passage 51 narrows towards the bottom end 5b in a narrowing portion 51d, for example a conically narrowing portion. The width of the opening 52 may be greater than the greatest outer diameter of the head 3, such that the head 3 may be inserted from the bottom end 5b into the accommodation space 53. To enable the insertion of the head 3 from the bottom end 5b when the pressure element 6 is in the receiving part 5, the width of the portions of the accommodation space 53 are such that the pressure element 6 can expand therein to permit the insertion of the head 3.

In the direction of the longitudinal axis L of the channel formed by the U-shaped recess 54 two opposite cutouts 57 are formed in the inner wall of the passage 51. The cutouts 57 may have a width in the circumferential direction such that a portion of the pressure element can extend therein and may be guided by the cutouts 57. In the axial direction, the cutouts 57 extend from a distance above the bottom 54a of the U-shaped recess 54 into the widening section 51b of the passage 51. An inner contour may be adapted to the outer contour of a portion of the pressure element as described in greater detail below. A depth of the cutouts 57 in the radial direction is at least as large so as to receive a portion of the pressure element therein. The cutouts 57 thus form radially enlarged sections that serve for permitting the insertion of the pressure element 6 from the bottom end 5b in a first rotational orientation relative to the receiving part 5.

A circumferential first groove 58a may be provided at the inner wall of the legs 55 at a distance from the bottom 54a of the U-shaped recess 54. The first inner groove 58a may provide a stop for restricting an upward movement of the pressure element 6 towards the top end 5a when the pressure element 6 is assembled with the receiving part 5 and when it is in an insertion position. Between the circumferential first groove 58a and the bottom of the U-shaped recess 54a in the axial direction there may be a circumferential second groove 58b for engagement with a portion of the pressure element 6 to secure a pre-locking position of the pressure element 6.

The receiving part 5 further may include two transverse holes 59 that extend completely through the legs 55, respectively, in a direction perpendicular to the central axis C and at a circumferential position approximately at the center of each of the legs 55. The through holes 59 may serve for accommodating the pins 7 as shown in Figs. 1 to 3. The pins 7 are configured to engage the pressure element to form a securing device to secure the pressure element 6 against rotation. In addition, the pins 7 may limit an upward movement of the pressure element 6.

Moreover, in the outer surface of each of the legs at a distance from the top end 5a circumferential tool engagement grooves 500 may be formed for engagement with a tool or an instrument. At an outer surface of the receiving part 5 aligned with the U-shaped recess two opposite flat areas 501 may be provided that reduce an overall width of the receiving part.

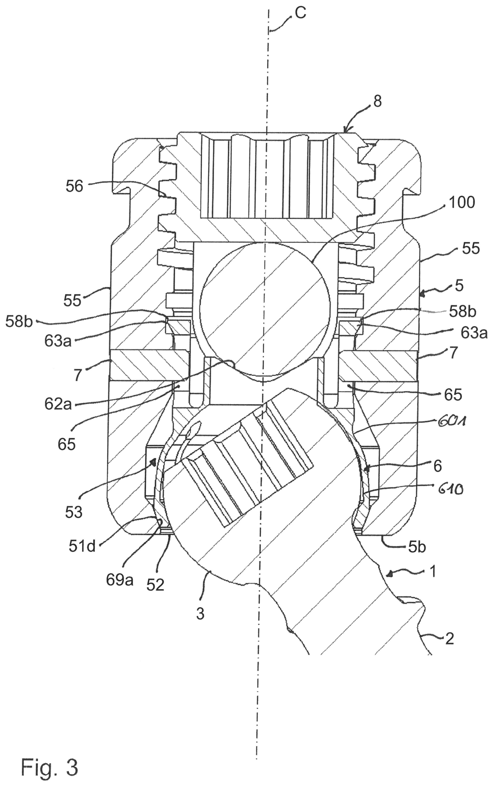

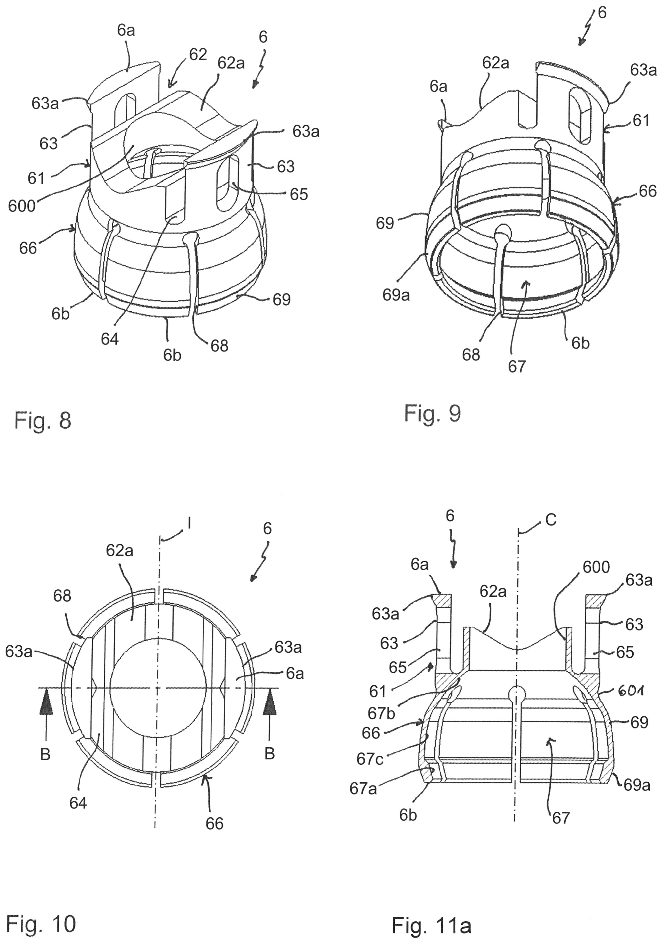

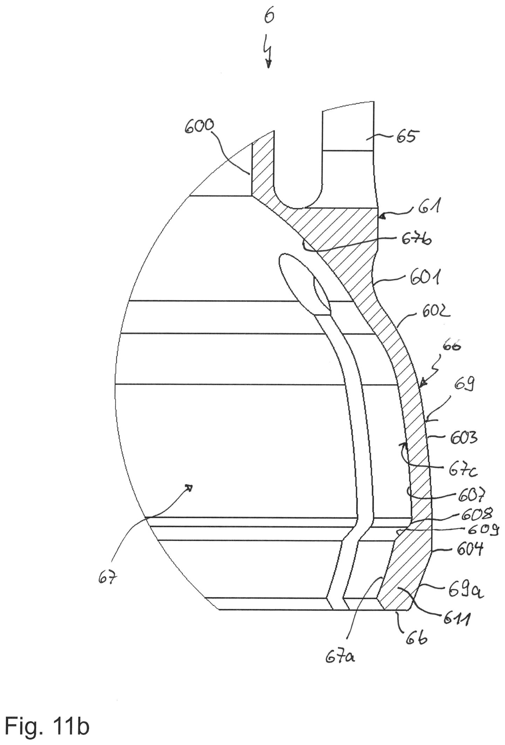

Referring further to Figs. 8 to 11a and 11b, the pressure element 6 will be described. Preferably, the pressure element 6 is a monolithic piece which is configured to be arranged in the passage 51 and to encompass the head laterally and from a free end of the head to exert pressure onto the head when the head 3 and the pressure element 6 are in the receiving part 5 such that the head 3 is pivotable with respect to the receiving part 5 and can be locked at an angle relative to the receiving part. In greater detail, the pressure element 6 has a first or top end 6a and a second or bottom end 6b. Adjacent to the top end the pressure element comprises a first portion 61 with a substantially cylindrical outer surface with an outer diameter that allows the pressure element 6 to move axially within the bore 51a of the passage 51 of the receiving part 5. At the top end 6a, a rod receiving recess 62 is formed that comprises a rod support surface 62a. The rod support surface 62a may have a substantially V-shaped cross-section with a longitudinal axis 1 extending substantially perpendicular to a cylinder axis of the first portion 61 that coincides with the central axis C of the receiving part 5 when the pressure element 6 is in the receiving part 5. The depth of the rod receiving recess 62 may be smaller than a diameter of the rod 100. Hence, when the rod 100 rests on the rod support surface 62a, the rod 100 projects over the top end 6a of the pressure element as shown, for example, in Fig. 3. The V-shape of the rod support surface 62a more easily facilitates use of rods with different diameters.

The rod receiving recess 62 is shaped such that two free upstanding legs 63 are formed that may be spaced apart from the rod support surface 62a on each side by a groove 64. By means of this, the legs 63 are slightly flexible in a direction transverse to the longitudinal axis 1 of the rod support surface 62a. A free end of each of the legs 63 may have a radially outwardly protruding rim 63a, an upper surface of which forms the top end 6a of the pressure element 6. The outwardly protruding rim 63a is configured to engage the respective cutout 57 of the receiving part 5 and may be guided therein. To secure an insertion position of the pressure element, the radially outwardly protruding rim 63a is configured to engage the first groove 58a provided at the inner surface of the legs 55 of the receiving part 5. Thus, the first groove 58a of the receiving part forms a first securing portion and the radially outwardly projecting rims 63a of the pressure element forms a second securing portion configured to cooperate with the first securing portion as explained in greater detail below. To secure a pre-locking position of the pressure element 6 in the receiving part 5, the radially outwardly protruding rim 63a is configured to engage the second groove 58b provided at the inner surface of the legs 55.

At the center of each of the legs 63 in a circumferential direction an axially extending elongate through hole 65 is provided, that serves for receiving the pins 7 as shown in Fig. 3.

A second portion 66 of the pressure element 6 has a substantially cap-like shape that is configured to receive the head 3 of the bone anchoring element 1. The second portion 66 of the pressure element comprises a hollow head receiving portion 67 with an opening at the bottom end 6b for inserting the head 3. The head receiving portion 67 may have a lower and an upper substantially spherical sections 67a, 67b that are shaped so as to matingly receive the spherical head 3. An intermediate section 67c may have a greater inner diameter for facilitating the insertion of the head 3. In addition, a plurality of axial slits 68 are formed that are open at the second end 6b and may have an enlarged, preferably rounded, end portion 68a. The slits 68 extend along an axial length that covers at least the intermediate section 67c which has the greatest inner diameter. In general, the number, shape and size of the slits are selected such that a desired flexibility is achieved that allows the expansion of the head receiving portion 63 when the head 3 is inserted through the bottom end 6b and compression around an inserted head 3. The size of the head receiving portion 67 may also be such that the head 3 can be held therein by friction prior to final locking of the head 3 in the receiving part 5.

An outer surface 69 of the second portion 66 of the pressure element 6 may be rounded and may have a greater outer diameter than the outer diameter of the cylindrical first section 61. Adjacent to the bottom end 6b, the outer surface 69 comprises a narrowing portion 69a, for example a conically narrowing portion, that is configured to engage the narrowing portion 51d at the bottom region of the accommodation space 53 of the receiving part 5. When the pressure element 6 and the head 3 of the bone anchoring element 1 are in the receiving part 5 and the narrowing portion 69a engages the narrowing portion 51d of the accommodation space, the head 3 is prevented from being removed through the lower opening 52 of the receiving part.

The pressure element 6 in addition comprises a coaxial opening or bore 600 that allows access to the tool engagement recess 4 of an inserted head 3 with a tool, such as a driver.

As can be seen in Fig. 11a and in an enlarged view in Fig. 11b, the second portion 66 of the pressure element comprises in its outer surface between the outer surface 69 and the cylindrical first section 61 a groove 601 preferably extending fully circumferentially. The groove 601 forms a transition between the first section 61 and the outer surface 69 of the second section 66 of the pressure element 6. The circumferential groove 601 has a smallest diameter in a direction perpendicular to the central axis C which is smaller than the outer diameter of the cylindrical first section 61 and smaller than an outer diameter of the outer surface 69. A contour of the groove 601 may be spherical in a cross-sectional view shown in Fig. 11a and 11b. The outer surface 69 may have adjacent to the groove 601 a first outer surface portion 602, preferably a spherical portion with a first radius, with protrudes farther outward than the cylindrical first section 61. The outer surface 69 continues from the first outer surface portion 602 into a second outer surface portion 603, preferably also a spherical portion with a second radius greater than the first radius, until an edge 604 between the second outer surface portion 603 and the narrowing portion 69a. The edge 604 may form the greatest outer diameter of the second portion 66 of the pressure element.

The intermediate section 67c of the inner surface of the head receiving portion 67 which is between the upper and lower spherical sections 67b, 67a, comprises a first portion 607 that widens from the upper spherical section 67b towards the bottom end 6b until a position 608 with a greatest inner diameter of the head receiving portion 67. Adjacent thereto the intermediate section 67c has a second portion 609 that narrows towards the lower spherical section 67a, preferably in a conical shape. By means of this, there is a gap 610 between an inserted head 3 and the inner wall of the head receiving portion 67 in the region of the intermediate section 67c as shown for example in Fig. 3. It shall be noted that the pressure element 6 has a wall portion 611 adjacent to the bottom end 6b which has an increased radial thickness due to the narrowing second portion 609 of the intermediate section 67c. This may result in an increased clamping force for clamping the head 3.

When the bone anchoring element has an enlarged head 3 that is insertable only from the bottom end 5b into the receiving part, the head receiving portion 67 of the pressure element must be large and flexible enough permit insertion and accommodation of such an enlarged head. With the groove 601 and the shape of the inner and outer surface of the second portion 66 of the pressure element, a sufficient flexibility may be achieved that allows the second portion 66 of the pressure element to spread when the head 3 is inserted. In addition, the mounting of the pressure element 6 in the receiving part 5 may be facilitated.

The parts and portions of the bone anchoring device may be made of any material, preferably, however, of titanium or stainless steel or a bio-compatible metal or metal alloy or plastic material. For bio-compatible alloys, a NiTi alloy, for example, Nitinol, may be used. Other materials that can be used are, for example, magnesium or magnesium alloys. Bio-compatible plastic materials include, for example, polyether ether ketone (PEEK) or poly-L-lactide acid (PLLA). The parts can be made of the same or of different materials from one another.

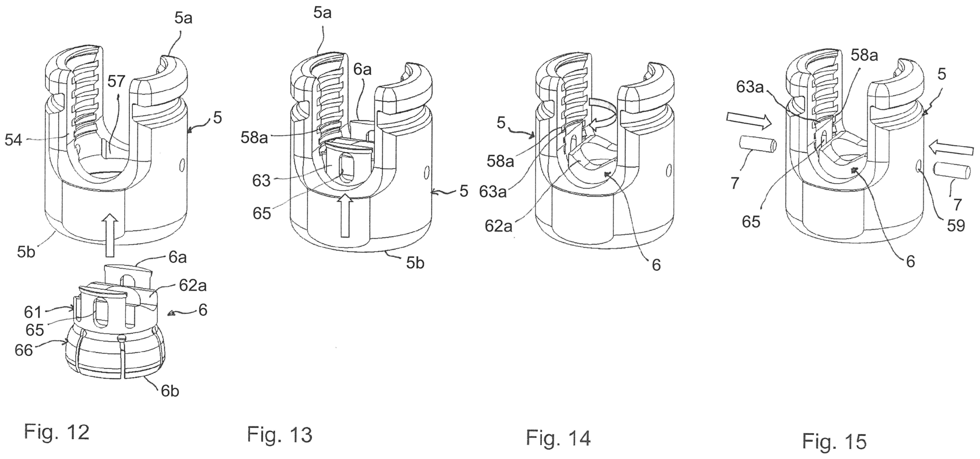

Referring to Figs. 12 to 15, steps of assembling the coupling device, i.e. the receiving part 5 with the pressure element 6, are shown. As depicted in Fig. 12, first, the pressure element 6 is oriented with its top end 6a facing towards the bottom end 5b of the receiving part 5 so that the pressure element 6 can be inserted from the bottom end 5b of the receiving part 5. The pressure element 6 assumes a first rotational orientation with respect to the receiving part 5 in which the longitudinal axis of the rod support surface 62a of the pressure element 6 and the longitudinal axis of the U-shaped recess 54 for receiving the rod are perpendicular.

Next, as shown in Fig. 13, the pressure element 6 is inserted into the receiving part 5 through the bottom end 5b in the first rotational orientation. During insertion, the outer rim 63a extends into the cutouts 57 which guide the pressure element 6 during insertion. The pressure element 6 is inserted up to an axial position in which the rim 63a on each leg 63 of the pressure element 6 has moved out of the cutout 57 and is at the axial height of the first groove 58a. The elongate recesses 65 may project out of the bottom 54a of the U-shaped recess to such an extent that the elongate recesses 65 can be engaged by the pins 7. Since the outer rim 63a extends axially out of the cutouts 57 once the pressure element 6 is fully inserted, the pressure element can be rotated.

Thereafter, as shown in Fig. 14, the pressure element 6 is rotated so that it assumes a second rotational orientation in which the longitudinal axis of the rod support surface 62a is aligned with the channel provided by the U-shaped recess 54. In the second rotational orientation, the pressure element 6 engages with the outer rim 63a the upper wall of the first groove 58a so that the pressure element is prevented from moving further towards the top end 5a of the receiving part 5 when the head 3 is inserted into the head receiving portion 67 of the pressure element 6. Thus, the upper groove 58a forms the first securing portion and the outer rim 63a forms the second securing portion that secure the pressure element 6 in the receiving part 5 in the insertion position for the head 3. As also shown in Fig. 14, the rod support surface 62a projects above the bottom 54a of the U-shaped recess 54.

Next, as shown in Fig. 15, the pressure element 6 is secured against inadvertent rotation by means of the pins 7 that are inserted into the pin holes 59. The pins 7 have such a length that they extend into the elongate recess 65. However, it may be possible to omit the pins 7 when the first portion 61 of the pressure element 6 has such a size that is secured in the portion 51a of the passage 51 by friction.

Usually, the coupling device is pre-assembled. In the clinical use, the bone anchoring device can be used in a first manner in which the bone anchoring element 1 is pre-assembled with the coupling device comprising the receiving part 5 and the pressure element 6 outside a patient's body or in a second manner in which the bone anchoring element 1 is already inserted in bone or in a vertebra and the coupling device is mounted *in-situ* on the head 3 of the bone anchoring element.

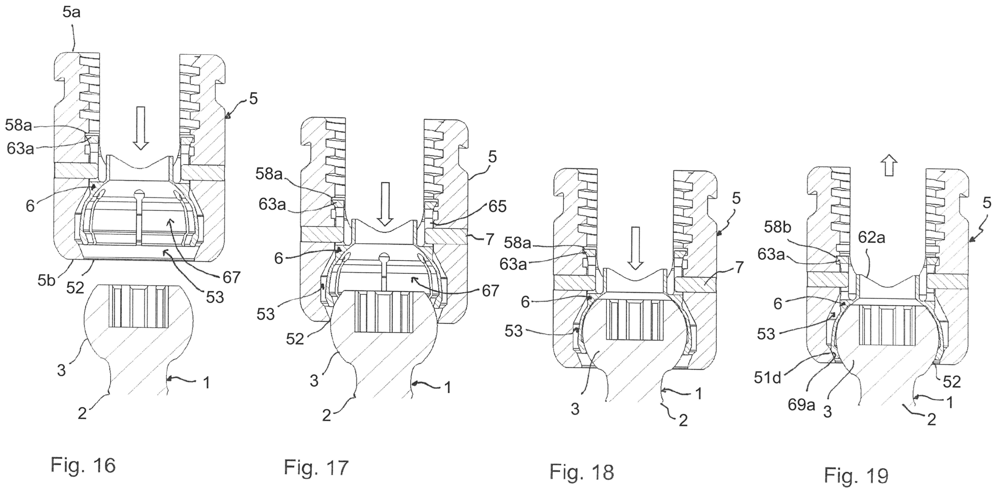

With reference to Figs. 17 to 19, the mounting of the coupling device to the bone anchoring element is described. As shown in Fig. 16, the pressure element 6 is in the insertion position in which the rim 63a is in the first groove 58a and the flexible second portion 66 of the pressure element is located in the widened sections 51b and 51c of the accommodation space 53.

As shown in Fig. 17, the coupling device is placed onto the head 3 of the bone anchoring element so that the head 3 enters through the lower opening 52 into the accommodation space 53 of the receiving part 5 and into the head receiving portion 67 of the pressure element 6. The pressure element 6 is prevented from moving towards the top end 5a of the receiving part as the top end 6a of the outer rim 63a abuts against the upper wall of the first groove 58a. Thus, the pressure element 6 is secured in the insertion position.

Next, as shown in Fig. 18, the pressure element 6 is pressed downward. As shown in Fig. 19, since the legs 63 of the pressure element 6 are slightly flexible, the outer rim 63 can disengage from the first groove 58a when the pressure element 6 is pressed down and engage the second groove 58b. By pulling the receiving part 5, the pressure element is moved further downward relative to the receiving part 5 until the narrowing outer surface portion 69 engages the narrowing inner surface portion 51d of the receiving part 5. Thus, as depicted in Fig. 19, the pressure element 6 is in a pre-locking position in which the lower opening 52 is reduced by the pressure element 6 so that the head 3 is prevented from being removed through the lower opening 52.

Due to the large outer diameter of the head 3, the bone anchoring element 1 can pivot in the receiving part to a greater maximum angle as compared to bone anchoring devices with smaller heads. For example, instead of heads with a greatest outer diameter of 7 mm, bone anchoring elements with a head of a greatest outer diameter of 8 mm can be used. The maximum pivot angle that the shank 2 can form with the central axis C may be 35° or more. Due to the large head, a stable and robust implant with increased holding force can be provided.

In use, once desired angular position of the bone anchoring element 1 relative to the receiving part 5 has been achieved, the rod 100 is inserted and the locking element 8 is inserted and tightened to lock the head and the rod.

In the clinical use, a plurality of polyaxial bone anchoring devices is inserted into bone parts or into vertebrae, in particular into the pedicles of vertebrae. The coupling devices are then aligned so that the rod can be received in the rod receiving portions of two or more of the bone anchoring devices.

Modifications of the above described embodiments are conceivable. In particular, the shape of the parts is not limited to the detailed shape shown in the figures. Deviations may be possible and encompassed by the disclosure. For example, the first and the second securing portions may be located at other positions on the receiving part and the pressure element and may be shaped differently. It may be sufficient to have only one first and only one second securing portion on the same side relative to the rod receiving recess.

Instead of the locking member being a set screw all other kinds of locking assemblies known in the art may be used. For the bone anchoring element, all types of bone anchoring elements are suitable for anchoring in bone or a vertebra, such as bone screws, bone nails, etc. may be used. The rod can be any elongate device that is configured to connect two bone anchoring devices. The rod may have various shapes and/or varying cross-section along its length. The rod may be stiff or more flexible.

The spherical outer surface portion of the head may be only partly spherical seen in the circumferential direction and the seat portion in the head receiving portion may be adapted thereto such that pivoting of the head is possible only in a predefined plane. Other restriction structures to limit the pivoting of the bone anchoring element to one or several distinct planes may also be applied to provide such a uniplanar bone anchoring device.

Moreover, the head receiving portion may have a design that allows to pivot the bone anchoring element to a greater pivot angle to one side compared to other sides.

## Claims

1. A coupling device for coupling a rod (100) to a bone anchoring element (1) having a head (3) and a shank (2),
the coupling device including
a receiving part (5) having a first end (5a), a second end (5b), a central axis (C) extending between the first end (5a) and the second end (5b) and a passage (51) extending from the first end (5a) to the second end (5b), a recess (54) for receiving the rod (100), the recess (54) extending from the first end (5a) towards the second end (5b) and forming two free legs (55), and an accommodation space (53) for accommodating the head (3) of the bone anchoring element (1), the accommodation space (53) having an opening (52) at the second end (5b),
a pressure element (6) configured to be located at least partially in the accommodation space (53) and comprising a first portion (61) comprising a rod support surface (62a) and a second portion (66) having a hollow interior (67) with an opening defining a flexible portion configured to accommodate and clamp the head (3),
wherein the receiving part (5) comprises a first securing portion (58a) and the pressure element (6) comprises a second securing portion (63a) configured to engage the first securing portion (58a),
wherein the pressure element (6) is insertable into the receiving part (5) from the second end (5b) in a first rotational orientation relative to the receiving part (5) in which the first securing portion and the second securing portion are out of engagement and wherein the pressure element (6) is rotatable in the receiving part (5) to assume a second rotational orientation with respect to the receiving part (5) in which the first securing portion (58a) and the second securing portion (63a) are engaged to prevent the pressure element (6) from moving towards the first end (5a).

2. The coupling device of claim 1, wherein the passage (51) comprises a portion (51a) along the central axis (C) with a smallest width and the pressure element (6) is sized to clamp a head (3) with a greatest width greater than the smallest width of the passage (51).

3. The coupling device of claim 1 or 2, wherein the second portion (66) of the pressure element (6) extends around an inserted head (3) at least in a region including a greatest outer diameter of the head (3), preferably wherein the second portion (66) of the pressure element (6) is sized such that it covers an inserted head (3) from a free end thereof and extends beyond a region including the greatest outer diameter of the head (3).

4. The coupling device of one of claims 1 to 3, wherein when the first securing portion (58a) and the second securing portion (63a) are engaged, the first securing portion (58a) provides a stop to prevent the removal of the pressure element (5a) through the first end.

5. The coupling device of one of claims 1 to 4 wherein the first securing portion (58a) is a groove and the second securing portion (63a) is a projection.

6. The coupling device of one of claims 1 to 5, wherein the second securing portion (63a) is provided at an outer surface of the first portion (61) of the pressure element (6), preferably wherein the first portion (61) of the pressure element (6) comprises two legs (63) wherein the second securing portion (63a) is provided at an outer surface of at least one of the legs (63).

7. The coupling device of one of claims 1 to 6, wherein the passage (51) comprises at least one radially enlarged section (57) between the accommodation space (53) and the first end (5a) that permits the second securing portion (63a) to extend therein when the pressure element (6) is in the receiving part (5) in the first rotational orientation, preferably
wherein a width of the radially enlarged section (57) in the radial direction is greater than a width of the passage (51) in a direction perpendicular thereto.

8. The coupling device of claim 7, wherein the radially enlarged section (57) is aligned with the recess (54) for receiving the rod (100) and/or
wherein the radially enlarged section (57) extends at least from a bottom (54a) of the recess (54) for receiving the rod into an upper portion (51b) of the accommodation space (53).

9. The coupling device of on of claims 7 or 8, wherein the enlarged section (57) has a size in the circumferential direction that is adapted to guide the second securing portion (63) during an axial movement of the pressure element (6).

10. The coupling device of one of claims 1 to 9, wherein in the first rotational orientation of the pressure element a longitudinal axis (1) of the rod support surface (62a) is substantially perpendicular, preferably is perpendicular, to a longitudinal axis of the recess (54) for receiving the rod (100) and more preferably wherein in the second rotational orientation the rod support surface (62a) is aligned with the longitudinal axis of the recess (54).

11. The coupling device of one of claims 1 to 10, wherein the receiving part (5) has near the opening (52) a narrowing portion (51d) configured to cooperate with a corresponding narrowing portion (69a) of the pressure element (6) to clamp the head.

12. The coupling device of one of claims 1 to 11, wherein between the first portion (61) and the second portion (66) of the pressure element (6) a groove (601), preferably a circumferential groove (601), is formed.

13. The coupling device of one of claims 1 to 12, wherein an inner wall of the hollow interior (67) of the pressure element comprises an upper portion (67b) and a lower portion (67a) mating a shape of an inserted head (3) and an intermediate portion (67c) and wherein a gap (610) is between the inserted head and the intermediate portion (67c).

14. A bone anchoring device comprising a coupling device of one of claims 1 to 13, further comprising an anchoring element (1) having a shank (2) and a head (3), preferably wherein the head (3) has a greatest outer diameter that is smaller than the opening (52) at the second end (5b) of the receiving part and greater than a smallest width of the passage (51).

15. A method for assembling a coupling device of one of claims 1 to 13, including the steps of
orienting the pressure element (6) relative to the receiving part (5) such that the pressure element has a first rotational orientation,
inserting the pressure element (6) in the first rotational orientation into the receiving part (5) from the second end (5b) of the receiving part (5), and
rotating the pressure element (6) to assume the second rotational orientation to engage the first securing portion (58a) and the second securing portion (63a).

## Amended claims

### Amended claims in accordance with Rule 137(2) EPC.

1. A coupling device for coupling a rod (100) to a bone anchoring element (1) having a head (3) and a shank (2),
the coupling device including
a receiving part (5) having a first end (5a), a second end (5b), a central axis (C) extending between the first end (5a) and the second end (5b) and a passage (51) extending from the first end (5a) to the second end (5b), a recess (54) for receiving the rod (100), the recess (54) extending from the first end (5a) towards the second end (5b) and forming two free legs (55), and an accommodation space (53) for accommodating the head (3) of the bone anchoring element (1), the accommodation space (53) having an opening (52) at the second end (5b),
a pressure element (6) configured to be located at least partially in the accommodation space (53) and comprising a first portion (61) comprising a rod support surface (62a) and a second portion (66) having a hollow interior (67) with an opening defining a flexible portion configured to accommodate and clamp the head (3),
wherein the receiving part (5) comprises a first securing portion (58a) and the pressure element (6) comprises a second securing portion (63a) configured to engage the first securing portion (58a), **characterized in that**
the pressure element (6) is insertable into the receiving part (5) from the second end (5b) in a first rotational orientation relative to the receiving part (5) in which the first securing portion and the second securing portion are out of engagement and wherein the pressure element (6) is rotatable in the receiving part (5) to assume a second rotational orientation with respect to the receiving part (5) in which the first securing portion (58a) and the second securing portion (63a) are engaged to prevent the pressure element (6) from moving towards the first end (5a).

2. The coupling device of claim 1, wherein the passage (51) comprises a portion (51a) along the central axis (C) with a smallest width and the pressure element (6) is sized to clamp a head (3) with a greatest width greater than the smallest width of the passage (51).

3. The coupling device of claim 1 or 2, wherein the second portion (66) of the pressure element (6) extends around an inserted head (3) at least in a region including a greatest outer diameter of the head (3), preferably wherein the second portion (66) of the pressure element (6) is sized such that it covers an inserted head (3) from a free end thereof and extends beyond a region including the greatest outer diameter of the head (3).

4. The coupling device of one of claims 1 to 3, wherein when the first securing portion (58a) and the second securing portion (63a) are engaged, the first securing portion (58a) provides a stop to prevent the removal of the pressure element (5a) through the first end.

5. The coupling device of one of claims 1 to 4 wherein the first securing portion (58a) is a groove and the second securing portion (63a) is a projection.

6. The coupling device of one of claims 1 to 5, wherein the second securing portion (63a) is provided at an outer surface of the first portion (61) of the pressure element (6), preferably wherein the first portion (61) of the pressure element (6) comprises two legs (63) wherein the second securing portion (63a) is provided at an outer surface of at least one of the legs (63).

7. The coupling device of one of claims 1 to 6, wherein the passage (51) comprises at least one radially enlarged section (57) between the accommodation space (53) and the first end (5a) that permits the second securing portion (63a) to extend therein when the pressure element (6) is in the receiving part (5) in the first rotational orientation, preferably
wherein a width of the radially enlarged section (57) in the radial direction is greater than a width of the passage (51) in a direction perpendicular thereto.

8. The coupling device of claim 7, wherein the radially enlarged section (57) is aligned with the recess (54) for receiving the rod (100) and/or
wherein the radially enlarged section (57) extends at least from a bottom (54a) of the recess (54) for receiving the rod into an upper portion (51b) of the accommodation space (53).

9. The coupling device of on of claims 7 or 8, wherein the enlarged section (57) has a size in the circumferential direction that is adapted to guide the second securing portion (63) during an axial movement of the pressure element (6).

10. The coupling device of one of claims 1 to 9, wherein in the first rotational orientation of the pressure element a longitudinal axis (1) of the rod support surface (62a) is substantially perpendicular, preferably is perpendicular, to a longitudinal axis of the recess (54) for receiving the rod (100) and more preferably wherein in the second rotational orientation the rod support surface (62a) is aligned with the longitudinal axis of the recess (54).

11. The coupling device of one of claims 1 to 10, wherein the receiving part (5) has near the opening (52) a narrowing portion (51d) configured to cooperate with a corresponding narrowing portion (69a) of the pressure element (6) to clamp the head.

12. The coupling device of one of claims 1 to 11, wherein between the first portion (61) and the second portion (66) of the pressure element (6) a groove (601), preferably a circumferential groove (601), is formed.

13. The coupling device of one of claims 1 to 12, wherein an inner wall of the hollow interior (67) of the pressure element comprises an upper portion (67b) and a lower portion (67a) mating a shape of an inserted head (3) and an intermediate portion (67c) and wherein a gap (610) is between the inserted head and the intermediate portion (67c).

14. A bone anchoring device comprising a coupling device of one of claims 1 to 13, further comprising an anchoring element (1) having a shank (2) and a head (3), preferably wherein the head (3) has a greatest outer diameter that is smaller than the opening (52) at the second end (5b) of the receiving part and greater than a smallest width of the passage (51).

15. A method for assembling a coupling device of one of claims 1 to 13, including the steps of
orienting the pressure element (6) relative to the receiving part (5) such that the pressure element has a first rotational orientation,
inserting the pressure element (6) in the first rotational orientation into the receiving part (5) from the second end (5b) of the receiving part (5), and
rotating the pressure element (6) to assume the second rotational orientation to engage the first securing portion (58a) and the second securing portion (63a).
